# FASCICULE DE BREVET EUROPEEN

(11) **EP 2 951 138 B1**
(45) Date de publication et mention de la délivrance du brevet: **30.06.2021**
(21) Numéro de dépôt: 14706883.7
(22) Date de dépôt: 04.02.2014
(51) Int. Cl.: C07C 1/24, C07C 6/04

(54) **PROCEDE DE PREPARATION D'UNE OLEFINE PAR CONVERSION CATALYTIQUE D'AU MOINS UN ALCOOL**
VERFAHREN ZUR HERSTELLUNG VON OLEFIN DURCH KATALYTISCHE UMWANDLUNG VON MINDESTENS EINEM ALKOHOL
METHOD FOR PRODUCING AN OLEFIN BY CATALYTIC CONVERSION OF AT LEAST ONE ALCOHOL

(30) Priorité: 04.02.2013 FR 1350926
(43) Date de publication de la demande: 09.12.2015
(73) Titulaire: Adisseo France S.A.S., 92160 Antony (FR); Centre National de la Recherche Scientifique (C.N.R.S.), 75794 Paris Cedex 16 (FR); Université Claude Bernard, 69100 Villeurbanne (FR)
(72) Inventeur: MILLET, Jean-Marc, 69006 Lyon (FR); BELLIERE-BACA, Virginie, 78570 Andresy (FR); DUDESERT, Thi Tuyet Nhung, 63400 Chamalières (FR); HUET, Robert, 75015 Paris (FR); REY, Patrick, 69003 Lyon (FR); AFANASIEV, Pavel, 69150 Decines (FR)
(74) Mandataire: Agasse, Stéphane
(86) Numéro de dépôt international: PCT/FR2014/050204
(87) Numéro de publication internationale: WO 2014/118484

(56) Documents cités:
- EP-A1- 2 157 074
- EP-A1- 2 157 074
- WO-A1-2011/115656
- GB-A- 317 500
- S S JEWUR ET AL: "The role of surface acidity of boron phosphate in the activity and selectivity of the dehydration of alcohols", JOURNAL OF CATALYSIS., vol. 57, 1979, pages 167-176, XP002715975, ACADEMIC PRESS, DULUTH, MN. ISSN: 0021-9517

## Description

La présente invention concerne un procédé catalytique de fabrication d'oléfines, de diènes, de polyènes par déshydratation d'alcools.

Les oléfines, encore appelés carbures éthyléniques ou alcènes, sont des hydrocarbures de formule générale CₙH₂ₙ avec n supérieur ou égal à 2. Les diènes et polyènes sont des hydrocarbures dont la molécule renferme deux liaisons éthyléniques ou plus. Entre dans cette définition générale, tout hydrocarbure ayant une ou plusieurs liaisons éthyléniques et comportant toute(s) fonction(s), par exemple amine primaire, secondaire, tertiaire, alcool primaire, secondaire, tertiaire, carbonyle dont aldéhyde, ..., conjuguées ou non avec la ou une desdites liaisons éthyléniques.

Les oléfines légères, telles que l'éthylène, le propène et les butènes, ainsi que le butadiène, sont d'importantes matières premières hydrocarbonées pour les synthèses chimiques et l'industrie pétrochimique. Leurs nombreuses réactions d'addition les placent au centre d'un ensemble de fabrications de gros tonnages d'intermédiaires fonctionnels. L'éthylène va par exemple, s'oxyder en époxyéthane (oxyde d'éthylène) ou bien être une source de glycol, d'éthanolamines, d'éthers et d'esters du glycol, s'oxyder en éthanal (acétaldéhyde), se chlorer et se déshydrochlorer en chlorure de vinyle, s'additionner au benzène puis se déshydrogéner en styrène ou encore se polymériser en polyéthylènes. Le propène permet d'accéder à une grande variété de produits pétrochimiques tels que le polypropène, l'acroléine, l'acrylonitrile, le cumène, les alcools oxo (butanol, 2-éthyl-butanol, 2-éthyl-hexanol), l'oxyde de propène, l'acide acrylique, l'alcool isopropylique, et les produits chimiques polygas. Utilisé comme synthon dans les unités d'alkylation, de polymérisation catalytique, et de dimérisation, il permet de produire des mélanges d'essences à indice d'octane élevé. Le propène et les butènes sont également transformés en produits intermédiaires fonctionnels. Leurs polymérisations et copolymérisations conduisent à des macromolécules présentant des propriétés pratiques remarquables. L'isobutylène, également connu comme l'isobutène ou le 2-méthylpropène, constituent des monomères de départ pour le caoutchouc butyle (copolymère d'isobutylène et de petites quantités d'isoprène). L'isoprène, aussi connu comme le 2-méthylbut-1,3-diène ou le terpène, est le précurseur du caoutchouc synthétique (polyisoprène), des copolymères séquencés styrène-isoprène-styrène, et du caoutchouc butyle. Le buta-1,3-diène, souvent directement appelé butadiène, est principalement utilisé dans la fabrication de caoutchouc synthétique, de vernis, du nylon et des peintures au latex (botaniques). Un grand nombre de pneus de voiture sont fabriqués avec du caoutchouc buna, copolymère du butadiène et du styrène. Le butadiène est aussi le principal réactif pour la synthèse du chloroprène par chloration suivie d'une isomérisation et d'une déshydrochloration. Ce diène est également utilisé pour produire l'adiponitrile et l'hexaméthylènediamine par réaction avec l'acide cyanhydrique. Plusieurs procédés utilisent le butadiène pour produire le butan-1,4-diol. Le butadiène est un excellent réactif pour la réaction de Diels-Alder et permet la synthèse du 4-vinylcyclohexène (réactif pour la production du styrène), du 1,5-cyclooctadiène et du 1,5,9-cyclodecatriène.

A l'heure actuelle, ces matières premières et intermédiaires de synthèse sont principalement obtenus à partir de combustibles fossiles par vapocraquage, craquage catalytique, déshydrogénation catalytique et métathèse. Ces procédés consomment beaucoup d'énergie et émettent beaucoup de CO₂, gaz à effet de serre.

La principale source d'éthylène, pilier de l'industrie chimique mondiale, est le vapocraquage de produits pétroliers liquides (naphta ou gazole) ou de liquides de gaz naturel (éthane, propane et butane) où il est accompagné de ses premiers homologues : le propène ou propylène, le but-1-ène et les but-2-ènes cis et trans, l'isobutylène et le but-1,3-diène et dont il est séparé par distillation fractionnée.

Le propène, comme l'éthylène, est une pierre angulaire de l'industrie pétrochimique. Aujourd'hui, le propène provient exclusivement de combustibles fossiles. Il s'agit du co-produit majoritaire dans la fabrication d'éthylène par craquage thermique. Les unités de vapocraquage du naphta ou du gazole sont plus sélectives en propène que celles qui utilisent le gaz de pétrole liquéfié comme matière première. Les raffineries de pétrole génèrent également d'importantes quantités de propène comme un sous-produit des opérations de fissuration, la cokéfaction et viscoréduction catalytiques. Le propène peut également être obtenu par déshydrogénation catalytique de propane ou par métathèse de l'éthylène et des but-2-ènes.

Les diènes et polyènes ne se trouvent que très rarement à l'état naturel. L'un des diènes les plus simples, l'isoprène CH₂=C(CH₃)-CH=CH₂, résulte de la pyrolyse de plusieurs terpènes et polyterpènes naturels. Les autres sont accessibles par de nombreuses synthèses. Le butadiène, présent lors du craquage des hydrocarbures (5 % de butadiène est produit dans le craquage des essences légères), est séparé du mélange par distillation des fractions C₄. L'obtention du butadiène pur n'est pas possible par simple distillation de cette fraction, car le butane et le butadiène forment un azéotrope. Cette séparation requiert généralement une extraction liquide-liquide ou une distillation extractive. Le butadiène est également synthétisé industriellement par déshydrogénation du butane, ou de mélanges de butènes et de butane.

Les récents développements technologiques permettent d'envisager une disponibilité à court terme de plus en plus importante d'alcools purs ou en mélanges, capables de réduire la dépendance en combustibles fossiles et d'atténuer les atteintes à l'environnement, notamment en termes d'émissions de dioxyde de carbone (voir le rapport EPA/600/R-07/144, Décembre 2007, biomass conversion : emerging technologies, feedstocks, and products ; SRI BIOTECHNOLOGY-BASED CHEMICALS by Hossein Janshekar, Kazuteru Yokose, Marifaith Hackett, and Xiaomeng Ma, SRI consulting report, CHEMICAL BUILDING BLOCKS FROM RENEWABLES, Marifaith Hackett, Septembre 2011).

Le méthanol est produit commercialement par réaction du gaz de synthèse sous pression en présence d'un catalyseur (voir CEH Marketing Research Report, METHANOL, Guillermo A. Saade, Juin 2009). Le gaz de synthèse est un mélange de gaz composé principalement d'oxyde de carbone et d'hydrogène, avec de petites quantités de dioxyde de carbone et d'autres gaz. Il est produit principalement à partir du gaz naturel mais également à partir d'autres sources d'hydrocarbures (naphta, résidus de pétrole, de charbon et, au moins potentiellement, les gaz des sites d'enfouissement contenant du méthane). Afin de limiter les coûts de production du méthanol, de nombreux sites industriels augmentent leurs capacités de production pour bénéficier de l'impact positif de facteur d'échelle, et d'autres orientent leurs recherches sur la réaction en une seule étape du méthane et de l'oxygène sans formation intermédiaire du gaz de synthèse.

Tous les produits issus de la biomasse, de quelqu'origine qu'elle soit, permettent de produire en direct ou comme sous-produits, par fermentation ou par voie chimique, des composés chimiques fonctionnalisés comme les alcools, diols, polyols en C6, C5 et C4, voire des synthons en C1, C2 et C3. On peut citer notamment un regain d'intérêt pour le butanediol biosourcé, incluant le butane-1,4-diol et le butane-2,3-diol. Un rapport récent publié par le cabinet américain Transparency Market Research tend à démontrer que le butane-2,3-diol biosourcé est en passe de remplacer sa version d'origine fossile. Ce diol pourrait offrir une solution alternative propre à des coûts compétitifs. L'un des grands débouchés du butane-2,3-diol biosourcé demeure la production de buta-1,3-diène ; matière première en très forte croissance. Ainsi, GB317500A décrit un procédé de préparation du butadiène par conversion du 1,3-butylène-glycol en butadiène, en présence d'un catalyseur à base de phosphate de cérium supporté sur de la pierre ponce. Autre exemple particulièrement connu, l'éthanol qui reste majoritairement produit par la fermentation d'un hydrate de carbone (amidon, sucre ou cellulose), suivie d'une distillation et de traitements appropriés à son utilisation finale (carburant, solvant, matières premières chimiques...). Ainsi, il est disponible commercialement soit pur (99,9% éthanol anhydre), soit en solution dans de l'eau en concentration supérieure à 80%. Beaucoup de recherches se concentrent sur le développement de procédés qui peuvent produire de l'éthanol à faible coût à partir de matières premières non alimentaires, c'est notamment le cas de l'éthanol cellulosique (voir CEH Marketing Research Report, ETHANOL, Eric Linak, Hossein Janshekar and Yoshio Inoguchi, Avril 2009).

La fermentation ABE (acétone-butanol-éthanol) est un procédé qui utilise la fermentation bactérienne anaérobique pour produire de l'acétone, du n-butanol et de l'éthanol à partir d'amidon. L'exploitation industrielle de la fermentation ABE a commencé en 1916 avec l'isolement Chaim Weizmann de *Clostridium acetobutylicum,* comme décrit dans le brevet US1315585A. Le procédé produit des mélanges d'acétone, de n-butanol et d'éthanol de rapports autour de 3-6-1. La matière première et la souche utilisées impactent directement la composition du mélange en sortie de fermenteur. Ainsi, par exemple, Cobalt Technologies, Inc décrit dans sa demande de brevet US2010330633A1 des rendements en butanol de 80% à partir de sucrose et de *Clostridium saccharubutylium* alors que Lanzatech New Zealand LTD, dans son brevet US8119844B2, décrit des rendements en n-butanol de 63% à partir de glycérol et de *Clostridium pasteurianum.* Afin de rendre la fermentation ABE rentable, de nombreux systèmes de récupération de produits *in-situ* ont été développés. Il s'agit notamment de distillation, pervaporation, extraction membranaire, adsorption et osmose inverse. Une valorisation encore plus pertinente viserait à exploiter directement le mélange obtenu.

Les directives européennes 2001/77/EC et 2003/30/EC, qui seront appliquées prochainement, projettent d'introduire 10% de Diester® (ou EMVH, Esters Méthyliques d'Huiles Végétales) dans les gazoles à l'horizon 2015. Ce biodiesel est fabriqué par transestérification des triglycérides contenus dans les liquides oléagineux, notamment les huiles végétales de palme, colza et tournesol, par du méthanol. Il coproduit approximativement, et suivant les procédés envisagés environ 100 kg de glycérol par tonne de Diester®. L'augmentation substantielle de la quantité de biodiesel qui va se produire au cours des prochaines années va générer des quantités importantes de glycérol équivalentes à plusieurs centaines de milliers de tonnes/an. Le glycérol ainsi produit, possède généralement une pureté de 75-90%. L'eau et les sels résiduels (provenant souvent des catalyseurs) sont les principaux contaminants de ce glycérol. Il sera plus ou moins raffiné en fonction de l'application à laquelle il est destiné. Quelques 1500 utilisations du glycérol sont déjà répertoriées, parmi lesquelles les suivantes illustrent à titre d'exemples sa présence dans de nombreuses et diverses formulations :
- hydratants en pharmacie (dans les suppositoires et les sirops) ou en cosmétologie dans les crèmes hydratantes, les savons à la glycérine, les dentifrices,
- solvants dans l'industrie alimentaire,
- plastifiants ou lubrifiants dans l'industrie chimique.

Ces applications s'avéreront nettement insuffisantes pour absorber les quantités de glycérol qui seront produites avec les biodiesels et bien qu'en progression, le marché conventionnel du glycérol (savons, pharmacie, ...) ne pourra pas non plus absorber un tel surplus. Il est donc vital de trouver de nouvelles applications permettant de valoriser de très gros volumes de glycérol. Fort de ce constat, de nombreux débouchés ont été étudiés ces dernières années (voir M. Pagliaro, M. Rossi: The Future of Glycerol, RSC Publishing, Cambridge (2008)), avec, en particulier, la conversion en propane-1,3-diol et en propane-1,2-diol, notamment utilisés comme monomères de base dans la synthèse des polyesters et polyuréthanes.

Dupont et Tate & Lyle ont développé une voie biofermentaire permettant d'accéder à partir d'amidon de maïs au propane-1,3-diol (enzyme co-développée avec Genencor (Danisco)) sans nécessairement passer par le glycérol.

Devant les récents développements qui devraient faciliter l'accès à de nombreux alcools, un regain d'intérêt se porte autour de la très ancienne réaction de Guerbet. Cette réaction permet d'obtenir des alcools à partir d'alcools plus courts à l'aide d'un catalyseur acido-basique. Dupont propose dans le brevet US7807857B2, une méthode pour obtenir des alcools de Guerbet (butanol et supérieurs) à partir d'éthanol. Virent Energy Systems décrit dans le brevet US7989664B2, une méthode d'obtention de polyols pouvant inclure des alcools tels que le méthanol, l'éthanol, l'alcool iso-propylique, le propanol, le butanol, le pentanol et l'hexanol. Kabushiki Kaisha Sangi, dans son brevet US8187347B2, propose une méthode permettant d'obtenir un mélange d'alcools et d'oléfines à partir d'alcools, et dans son brevet US8080695B2, décrit une méthode pour obtenir un mélange de butan-1-ol, d'hexanol, d'octanol et de décanol à partir d'éthanol.

Il faut souligner que la transformation de la biomasse n'est pas la seule route pour la production d'alcools en grande quantité. Ainsi les procédés syn-gaz sont modifiés pour conduire à des alcools autres que le méthanol, comme l'éthanol, les propan-1-ol et propan-2-ol et les butanols. Les catalyseurs sont à base de métaux du type Cu, Zn, Mo ou Cr, dopés avec des alcalins. Des procédés de production de mélanges d'alcools autres que le méthanol ont ainsi été développés par les sociétés Snamprogetti, Topsoe, Lurgi, Dow and IFP-Idemitsu. Les réactions qui ont lieu dans ces procédés incluent la réaction de gaz à l'eau, la beta Co addition, les homologations de l'éthanol et des autres alcools plus longs, la condensation, la déshydratation, la formation d'iso-alcools branchés et d'esters méthyliques.

Ainsi, certains alcools, comme le méthanol, l'éthanol et le butanol par exemple, sont déjà produits industriellement à grande échelle. Les technologies de demain permettront d'avoir accès à ces alcools et à bien d'autres alcools, purs ou en mélange à plus bas coût. Avec la demande croissante en oléfine, l'épuisement des matières premières fossiles traditionnelles et le développement de l'accès à des matières premières alternatives, la déshydratation sélective des alcools en oléfines apparaît comme une voie d'accès industrielle alternative prometteuse, d'autant plus qu'elle sera capable de traiter des mélanges d'alcools.

En règle générale, les réactions d'hydratation sont favorisées à basse température et les réactions de déshydratation à haute température. Pour obtenir les produits de déshydratation souhaités, il est donc nécessaire d'utiliser une température de réaction suffisante, et/ou un vide partiel pour déplacer l'équilibre de la réaction. La réaction de déshydratation peut être réalisée en phase liquide ou en phase gaz. Ce type de réaction est connu pour être catalysé par des acides inorganiques ou par des solides acides.

Beaucoup de catalyseurs acides sont efficaces dans la déshydratation de l'éthanol. Ces catalyseurs sont, pour la plupart, basés sur de l'alumine dopée, de l'acide phosphorique supporté, des silices-alumines ou des zéolithes (voir A. Morschbacker, Journal of Macromolecular Science, Part C: Polymer Reviews, 49 (2009) 79-84 ; O. Winter, E. Ming-Teck, Hydrocarb. Process Nov (1976) 125-133 ; C.B. Phillips, R. Datta, Ind. Eng. Chem. Res. 36 (1997) 4466-4475 ; T.M. Nguyen, R.L.V. Mao, Appl. Catal. 58 (1990) 119-129, X. Zhang, R. Wang, X. Yang, F. Zhang, Micro and Meso Materials 116 (2008) 210-215.). Les premiers catalyseurs industriels proposés étaient pratiquement tous basés sur de l'acide phosphorique supporté mais ils ont été rapidement supplantés par des alumines ou des silices-alumines en raison de leur plus forte productivité et de l'absence de corrosion inhérentes à leur utilisation.

Néanmoins ces derniers catalyseurs requièrent des températures de réaction élevées (430-450°C) et sont moins efficaces quand les solutions d'alcools traitées contiennent des quantités d'eau élevées. Pour ces raisons, des zéolithes synthétiques comme la HZSM5 ont été développées en tant que catalyseur [voir C.B. Phillips, R. Datta, Ind. Eng. Chem. Res. 36 (1997) 4466-4475 et T.M. Nguyen, R.L.V. Mao, Appl. Catal. 58 (1990) 119-129]. Elles ont été utilisées uniquement pour la déshydratation de l'éthanol et elles permettent d'abaisser la température de réaction (300°C) sans perte d'efficacité (par exemple une sélectivité de 95% en éthylène pour une conversion d'éthanol de 98% peut être obtenue sur un catalyseur à base de HZSM5). Des zéolithes du type SAPO-34 ont également été utilisées avec succès. Cependant la déshydratation des alcools a lieu sur les sites acides faibles ou de force moyenne du catalyseur et la présence de sites acides forts répartis de façon non uniforme, comme c'est le cas dans ces matériaux zéolithiques, induit la formation de produits non désirés et à un cokage important du catalyseur (voir X. Zhang, R. Wang, X. Yang, F. Zhang, Micro and Meso Materials 116 (2008) 210-215). Les zéolithes doivent donc être modifiées par dopage avec du phosphore (voir D.S. Zhang, R.J. Wang, X.X. Yang, Catal. Lett. 124 (2008) 384-391 et K. Ramesh, L.M. Hui, Y.F. Han, A. Borgna, Catal. Commun. 10 (2009) 567-571), des terres rares (voir J. Ouyang, F.X. Kong, G.D. Su, Y.C. Hu, Q.L. Song, Catal. Lett. 132 (2009) 64-72; US4873392A ; N. Zhan, Y. Hu, H. Li, D. Yu, Y. Han, H. Huang, Catal. Comm. 11 (2010) 633-637) ou encore dans le cas de la SAPO-34 par du nickel (voir X. Zhang, R. Wang, X. Yang, F. Zhang, Micro and Meso Materials 116 (2008) 210-215) pour éliminer les sites acides trop forts et augmenter le nombre de sites acides modérément forts ou même faibles.

Ces modifications améliorent la sélectivité et la stabilité des catalyseurs mais présentent néanmoins un défaut lié à la nécessité d'utiliser une vitesse massique horaire relativement faible.

Alors que la déshydratation du propanol-2 est utilisée couramment pour caractériser l'acidité de tous types de catalyseurs, la déshydratation du n-propanol n'a fait l'objet que de très peu d'études. Les meilleurs catalyseurs obtenus donnent un rendement de 100% à 380°C (voir P. Brandao, A. Philippou, J. Rocha, M.W. Anderson, Catal. Letters 80 (2002) 99) sur des catalyseurs à base de zéolithe.

La recherche sur la production de butanol par fermentation s'est intensifiée ces dernières années et d'importantes avancées ont été faites. Ces avancées concernent à la fois les souches microbiennes et la technologie de fermentation, la qualité des produits de départ qui peuvent être des résidus de biomasse et les techniques de séparation des produits obtenus. [A.P. Mariano, R. Maciel Filho, J. Bioenerg. Res. 5 (2012) 504. Et V. Menon, M. Rao, Progress in Energy and Combustion Science 38 (2012) 522]. Le Bio-butanol est désormais considéré comme un biocarburant possible car il peut être produit à l'échelle du million de tonnes.

Beaucoup d'applications ont été développées pour valoriser ce bio-butanol. Il peut être catalytiquement déshydrogéné ou déshydraté. Dans le premier cas on produit la butanone (ou méthyléthylcétone) qui est un solvant usuel de l'industrie chimique [C.F. Turner, J.W. McCreery, The Chemistry of Fire and Hazardous Materials. Boston, Massachusetts: Allyn and Bacon, Inc. (1981) 118.]. Dans le deuxième cas on produit les différents isomères du butène (but-1-ène, cis-but-2-ène, trans-but-2-ène et isobutène). La répartition de ces différents butènes dépend de l'alcool de départ (butan-1-ol, butan-2-ol et isobutanol), de la température (équilibre thermodynamique) et du catalyseur. Les butènes sont utilisés dans la fabrication de plastiques comme le but-1-ène ou de lubrifiants comme le but-2-ène ou de gommes (butyl rubber) et de méthyl tert-butyl éther (MTBE), ou d'iso-octane comme l'isobutène.

Le but-2-ène peut également être utilisé pour produire du propène par réaction de métathèse avec l'éthylène. Cette production par métathèse est amenée à se développer dans un futur proche car elle offre un accès à du propène totalement biosourcé. Toutefois, alors que le produit principal de la plupart des procédés de production de butanol par fermentation est le butan-1-ol, le butène nécessaire pour la réaction de métathèse est le but-2-ène. Il est donc important de disposer d'un procédé catalytique de conversion du butan-1-ol sélectif en but-2-ènes. Aucun catalyseur ne permettant d'obtenir une sélectivité acceptable n'a été reporté à notre connaissance.

Une avancée scientifique fondamentale consisterait à développer un catalyseur permettant la déshydratation sélective du butan-1-ol en but-2-ène en combinaison avec la déshydratation de l'éthanol en éthylène, ce catalyseur pouvant être utilisé et convertir sélectivement les deux alcools à la même température. De plus, si le catalyseur était également sélectif pour convertir le butan-2-ol en but-2-ènes, ni une séparation des alcools ni un enrichissement en l'un ou l'autre ne serait nécessaire. La déshydratation du mélange d'alcools pourrait se faire en une seule étape et conduire directement à un mélange optimal pour la réaction de métathèse.

Si un intérêt particulier existe à convertir simultanément l'éthanol et le butanol sur le même catalyseur, il peut également y avoir un intérêt à convertir d'autres mélanges d'alcools binaires ou plus complexes simultanément sur le même catalyseur, à la même température pour obtenir très sélectivement un mélange d'alcènes en évitant toutes réactions parasites de craquage ou de formation d'éthers. Le catalyseur utilisé dans une telle réaction devra être très actif et sélectif pour déshydrater tous les alcools considérés sur la même plage de température.

EP2157074A1 décrit un procédé de déshydratation du glycérol en acroléine en présence d'un catalyseur à base de sel cristallisé d'acide phosphorique et d'un lanthanide (terre rare), tels que Y, La, Ce, Pr et Nd. WO 2011/115656 décrit un procédé de déshydratation du propanol en propène en présence d'au moins un catalyseur Y2O3.

L'objet de la présente invention réside dans la mise en œuvre de catalyseurs robustes, actifs, sélectifs et régénérables, permettant de produire des oléfines, des diènes et/ou des polyènes à partir d'alcools, purs ou en mélange, selon une réaction de déshydratation, en vue d'obtenir le propène. Les inventeurs de la présente invention ont développé des catalyseurs très sélectifs, régénérables et actifs à plus basse température que les catalyseurs décrits dans l'art antérieur pour déshydrater des alcools.

Ainsi, l'invention concerne un procédé de préparation du propène comprenant la préparation d'une ou plusieurs oléfines, diènes ou polyènes, par conversion catalytique d'au moins un alcool possédant une chaîne carbonée d'au moins trois atomes de carbone et étant différent du propan-2-ol, en présence d'au moins un catalyseur à base d'au moins un phosphate d'un métal ou de plusieurs métaux M, M étant choisi parmi les 15 lanthanides (lanthane, praséodyme, néodyme, prométhium, samarium, europium, gadolinium, terbium, dysprosium, holmium, erbium, thulium, ytterbium, lutécium), l'yttrium et le scandium. Plus précisément, selon le procédé de l'invention :
- on prépare le propène par ladite conversion catalytique d'un alcool choisi parmi le propan-1-ol, éventuellement en mélange avec le propan-2-ol ;
- on prépare un butène par ladite conversion catalytique d'un alcool choisi parmi le butan-1-ol, le butan-2-ol et leurs mélanges, puis on convertit le butène en propène par métathèse ;
- on prépare un mélange éthylène et butène par ladite conversion catalytique de l'éthanol et d'au moins un autre alcool choisi parmi le butan-1-ol, le butan-2-ol et leurs mélanges, puis on convertit le mélange éthylène et butène en propène par métathèse.

Par alcool selon la présente invention, on entend une chaîne hydrocarbonée saturée ou insaturée, linéaire ou ramifiée, qui porte un ou plusieurs groupes hydroxy, comme les diols et tous autres polyols.

La présente invention est ci-après décrite plus en détails, faisant ressortir les variantes et avantages du procédé de l'invention, ainsi que ses applications.

De préférence, le catalyseur est à base d'au moins un phosphate d'un ou de plusieurs métaux M choisis parmi le lanthane, le praséodyme, le néodyme, le prométhium, le samarium, l'europium, le gadolinium, le terbium, le dysprosium, l'holmium, l'erbium, le thulium, l'ytterbium, le lutécium, l'yttrium et le scandium

Selon une mise en œuvre encore préférée, le catalyseur est à base d'au moins un phosphate d'un ou de plusieurs métaux M choisis parmi le lanthane, le néodyme, le gadolinium et le samarium.

Le catalyseur de l'invention peut être dopé par au moins un métal alcalin choisi parmi le césium, le rubidium et le potassium et/ou un métal de transition choisi dans les groupes 4 à 7.

Le rapport molaire P/M varie avantageusement de 0,5 à 2, et mieux encore de 0,9 à 1,5, M représentant le métal ou la somme des métaux M constitutifs dudit catalyseur.

Le procédé de l'invention présente un intérêt majeur en ce qu'il permet de traiter un ou des alcools purs, partiellement purifiés, non purifiés ou sous forme de compositions azéotropiques, ainsi que tout mélange de ceux-ci. Ils peuvent être traités sous gaz inerte en présence ou non d'un gaz oxydant (O₂, H₂O, CO₂, O₃...). La présence d'un tel gaz permet d'améliorer la stabilité du catalyseur dans le temps. Le ou les alcools peuvent être en solution aqueuse, de préférence, en une concentration d'au moins 1% en poids.

En raison de leur disponibilité et/ou de leur produit de déshydratation, des alcools préférés sont choisis parmi le propan-1-ol, butan-1-ol, le butan-2-ol, l'isobutanol, le but-3-ène-1-ol, le but-2-ène-1-ol, but-3-ène-1,2-diol, le butane-2,3-diol, le butane-1,3-diol, le butane-1,4-diol, le glycérol, le propane-1,2-diol, le propane-1,3-diol, l'érythritol, et leurs mélanges.

Selon l'invention, l'oléfine, diène ou polyène préparé en vue d'obtenir le propène possède une chaîne carbonée du même nombre d'atome de carbone que l'alcool ou les alcools à partir desquels ils sont préparés.

Un autre objet intéressant de l'invention est la mise en œuvre du procédé décrit précédemment pour la préparation d'un mélange d'oléfines, diènes et/ou polyènes en vue de d'obtenir le propène.

Il est possible de préparer un mélange d'au moins deux oléfines, diènes et/ou polyènes, à partir d'au moins un alcool possédant une chaîne carbonée d'au moins trois atomes de carbone et différent du propan-2-ol. Ainsi, il est possible de préparer un mélange d'au moins deux oléfines, diènes et/ou polyènes, à partir d'un mélange d'alcools comprenant au moins un alcool possédant une chaîne carbonée d'au moins trois atomes de carbone et différent du propan-2-ol et tout autre alcool.

La préparation desdites oléfines peut être réalisée par conversion catalytique d'un alcool ou d'un mélange d'alcools, en présence du même catalyseur ou de catalyseurs différents.

Ils peuvent être préparés dans le même réacteur, en présence du même catalyseur ou de catalyseurs différents ; ils peuvent aussi être fabriqués dans des réacteurs différents, en présence d'un catalyseur identique ou de catalyseurs différents.

Les catalyseurs de l'invention se montrent capables de transformer plusieurs alcools sélectivement alors que ceux-ci sont en mélange.

Avantageusement, le ou lesdits catalyseurs pour la préparation de ces oléfines/diènes/polyènes comprennent une phase active et un support et/ou un liant. Ils peuvent aussi être mis en forme, éventuellement en présence d'un liant. Ils peuvent aussi être régénérés.

Les alcools purs ou en mélange peuvent être traités sous gaz inerte en présence ou non d'un gaz oxydant (O₂, H₂O, CO₂, O₃...). La présence de tel gaz permet d'améliorer la stabilité du catalyseur dans le temps.

Par rapport à l'art antérieur, on apporte, selon l'invention décrite, un procédé impliquant la préparation d'oléfines en C₄ plus actif et plus sélectif en but-2-ène qu'en but-1-ène par déshydratation catalytique d'alcools en C₄ en présence d'un catalyseur qui, tout en permettant de convertir la totalité de l'alcool en C₄ de départ, peut être très facilement régénéré et possède une longue durée de vie. La réaction peut être réalisée en phase liquide, en phase gaz ou en milieu mixte bi-phasique.

Dans le cas d'une réaction en phase gazeuse, après déshydratation, le gaz de réaction est refroidi dans une colonne de trempe à l'eau, qui sépare la majeure partie des produits de l'eau et de l'alcool n'ayant éventuellement pas réagi. Le traitement ultérieur du courant d'oléfine, de diène et/ou de polyène brut dépend du niveau de pureté requis dans le produit final, notamment en fonction de l'utilisation envisagée.

Cette alternative permet ainsi de disposer d'un procédé compétitif de synthèse d'oléfines, de diènes et/ou de polyènes ou cétones moins dépendant des ressources fossiles traditionnelles, tout en atténuant les atteintes à l'environnement. Les récents développements technologiques et biotechnologiques laissent augurer, à brève échéance, une disponibilité accrue d'alcools purs ou en mélanges. Cet aggiornamento scientifique permettra d'abaisser les atteintes à l'environnement en réduisant les émissions des gaz à effet de serre, le dioxyde de carbone tout particulièrement.

Des exemples d'applications d'intérêt fondamental d'un procédé de l'invention sont ci-après exposés :
La préparation sélective de but-2-ène à partir de butan-1-ol, de butan-2-ol et de leurs mélanges;
La fabrication de propène, mettant en œuvre l'un quelconque des procédés suivants :
   - Préparation de propène par un procédé de l'invention, dans lequel l'alcool est choisi parmi le propan-1-ol, éventuellement en mélange avec le propan-2-ol ;
   - Préparation d'un butène par un procédé de l'invention, dans lequel l'alcool est choisi parmi le butan-1-ol, le butan-2-ol et leurs mélanges, puis conversion du butène en propène par métathèse ;
   - Préparation d'un mélange éthylène et butène par un procédé de l'invention, dans lequel au moins un alcool est l'éthanol et un autre alcool est choisi parmi le butan-1-ol, le butan-2-ol et leurs mélanges, puis conversion du mélange éthylène et butène en propène par métathèse.
La fabrication du butadiène mettant en œuvre un procédé de l'invention, dans lequel l'alcool est choisi parmi le but-2-ène-1-ol, le but-3-ène-1-ol, le but-3-ène-1,2-diol, le butane-2,4-diol, le butane-2,3-diol, butane-1,3-diol et le butane-1,4-diol et leurs mélanges ;
La fabrication d'isobutène à partir d'isobutanol ;
La fabrication de pentène et d'isoprène à partir d'alcools dont diols correspondants ;
La fabrication de l'acroléine, de l'acide acrylique, de l'acrylonitrile et du polypropylène à partir du propène produit selon la présente invention ;
La fabrication de l'aldéhyde-3-(méthylthio)propionique MMP, du 2-hydroxy-4-méthylthiobutyronitrile (HMTBN), de la méthionine, de l'acide 2-hydroxy-4-méthylthiobutanoïque (HMTBA), des esters de ce derniers, ou du 2-oxo-4-méthylthiobutanoïque (KMB), à partir d'acroléine, telle que fabriquée ci-dessus, ou à partir de propène.

La méthionine, le HMTBA et les esters de celui-ci et analogues, sont utilisés en nutrition animale et, dans leurs procédés industriels de synthèse, l'acroléine est généralement obtenue par oxydation du propène et/ou du propane. L'oxydation du propène en acroléine par l'air en présence d'eau est partielle, et le produit brut résultant, à base d'acroléine, contient aussi du propène et du propane n'ayant pas réagi, de l'eau et des sous-produits de la réaction d'oxydation, notamment des acides, aldéhydes et alcools.

L'acroléine et l'acide acrylique sont traditionnellement produits par oxydation ménagée en phase gazeuse du propène par l'oxygène de l'air en présence de catalyseurs à base d'oxydes de molybdène et/ou bismuth. L'acroléine ainsi obtenue peut soit être directement intégrée dans un procédé de fabrication d'acide acrylique, d'acrylonitrile, soit être utilisée comme intermédiaire de synthèse.

Les marchés de l'acroléine, l'un des plus simples des aldéhydes insaturés, de l'acrylonitrile, et de l'acide acrylique sont colossaux car ces monomères entrent dans la composition de nombreux produits de masse.

Par ailleurs, l'acroléine, composé hautement réactif de par sa structure, trouve de nombreuses applications, notamment comme intermédiaire de synthèse. Comme dit précédemment, elle est tout particulièrement utilisée comme intermédiaire clé entrant dans la synthèse de la D,L-méthionine et de son dérivé hydroxy-analogue, l'acide 2-hydroxy-4méthylthiobutanoïque (HMTBA). Ces additifs alimentaires sont massivement employés car ils entrent dans la composition de compléments alimentaires indispensables à la croissance des animaux (volailles, porcs, ruminants, poissons, ...).

Dans un certain nombre de cas, il peut être profitable de pouvoir augmenter, voire d'assurer les capacités de production des unités industrielles existantes en diversifiant la matière première engagée. Il apparaît donc tout particulièrement intéressant de pouvoir augmenter la productivité en acroléine, tout en réduisant la dépendance vis-à-vis de cette ressource issue du pétrole qu'est le propène et d'atténuer les atteintes à l'environnement. Le propène pourrait ainsi être obtenu par métathèse de l'éthylène et de n-butènes, obtenus seuls ou en mélanges par déshydratation des alcools ou directement par déshydratation du propan-1-ol - appelé aussi n-propanol ou par craquage d'oléfines supérieures.

L'obtention de propène par métathèse de l'éthylène et de n-butènes peut se schématiser selon le bilan suivant :

Ethylène + But-2-ène => Propène

Elle est favorable thermodynamiquement mais cette réaction est équilibrée. L'utilisation d'un excédant d'éthylène permet de favoriser la formation de propène. Cette réaction demeure sélective mais exige l'absence de toutes impuretés organiques. Il faut donc assurer une purification préalable des réactifs ou partir de réactifs hautement purifiés. C'est une réaction qui ne fonctionne qu'avec le but-2-ène. Une réaction parasite entre deux molécules de but-1 -ène fournirait du hex-3-ène et de l'éthylène. Il est donc soit nécessaire d'ajouter un réacteur d'isomérisation qui transforme le but-1-ène non réactif en une partie de but-2-ène, soit d'utiliser une coupe préalablement enrichie en but-2-ène. La production de propène avec une bonne sélectivité et un bon taux d'avancement sont favorisés par un excès de but-2-ène par rapport au but-1-ène et un excès d'éthylène. La demande de brevet de LUMMUS US2005/0124839A1 recommande par exemple de travailler avec un rapport molaire en réactif « éthylène/somme des butènes» compris entre 0,9 et 2. Le procédé Meta-4 de l'IFP propose un procédé continu de métathèse de production de propène à partir d'éthylène et de but-2-ène à basse température en phase liquide en présence d'un catalyseur hétérogène à base de rhénium (voir US6075173A). Une conversion équilibrée de 63% à 35°C est annoncée.

Une application très avantageuse de cette invention réside dans la synthèse d'une coupe enrichie en but-2-ène ou d'un flux favorable pour la production sélective de propène par métathèse d'éthylène et du butène.

Par rapport à l'art antérieur, on apporte, selon l'invention décrite, un procédé de préparation d'oléfines en C₄ plus sélectif en but-2-ène qu'en but-1-ène par déshydratation catalytique d'alcools en C₄ en présence d'un catalyseur qui, tout en permettant de convertir la totalité de l'alcool en C₄ de départ, peut être très facilement régénéré et présente une durée de vie significative.

De même, selon l'invention décrite, il est possible d'obtenir un mélange d'éthylène et de butènes favorisant la production de propène par métathèse, par déshydratation catalytique d'un mélange d'éthanol et d'alcool en C₄, et préférentiellement de butan-1-ol.

Le catalyseur peut être préparé de diverses façons (coprécipitation, synthèse hydrothermale...), bien connues de l'homme du métier. Ce dernier peut notamment se référer aux articles suivants, M. Anbia, M.K. Rofouel, S.W. Husain, Chin. J. Chem. 24 (2006) 1026-1030; J.A. Diaz-Guillen, A.F. Fuentes, S. Gallini, M.T. Colomer, J. Alloys and Compounds 427 (2007) 87-93; K. Rajesh, P. Shajesh, O. Seidel, P. Mukundan, K.G.K. Warrier, Adv. Funct. Mater. 17 (2007) 1682-1690.

Le précurseur de phosphore initial peut être choisi parmi plusieurs composés comme les phosphates d'ammonium et hydrogéno-phosphates d'ammonium, les phosphates alcalins préférentiellement de sodium, les acides phosphoriques préférentiellement orthophosphorique, l'oxyde de phosphore anhydride ou les composés organiques du phosphore comme les éthers phosphoriques.

Le catalyseur défini précédemment peut en outre répondre aux caractéristiques préférentielles ci-dessous, considérées seules ou en combinaison :
- les catalyseurs sont constitués majoritairement des phosphates et phosphates mixtes précédemment définis qui constituent la ou les phases actives principales des catalyseurs
- les catalyseurs sont principalement composés d'une phase orthophosphate pure ou en mélange
- le rapport molaire P/M varie de 0,5 à 2, plus avantageusement, il varie de 0,9 à 1,5, M représentant le métal ou la somme des métaux M constitutifs dudit catalyseur.

Le dit catalyseur peut également comprendre la phase active à base de phosphate et au moins un liant ou un support pour cette phase active. Le support ou le liant peut être constitué de silice pure (SiO₂), d'un silicate (d'alcalin, d'alcalino-terreux ou de terres rares) éventuellement en mélange entre eux ou avec les argiles, l'oxyde de titane (TiO₂), l'oxyde de bore (B₂O₃) ou des résines (sulphoniques, perfluorosulphoniques ou autres). Les liants ou supports préférés sont à base de silice, sous toutes ses formes connues de l'homme du métier, d'oxyde de titane et de leurs mélanges. La teneur pondérale en liant ou support du catalyseur est comprise entre 0 et 80%, plus particulièrement entre 5% et 50%.

Le support peut être préparé par mise en forme en présence ou en absence de liant par toute technique connue de l'homme du métier. La mise en forme peut être réalisée par exemple par extrusion, par pastillage, par la méthode de la coagulation en goutte (oil-drop), par granulation au plateau tournant ou par toute autre méthode bien connue de l'homme du métier. Au moins une calcination peut être effectuée après l'une quelconque des étapes de la préparation, elle est habituellement effectuée sous air à une température d'au moins 150°C, de préférence au moins 300°C.

Comme dit précédemment, le catalyseur de l'invention présente l'intérêt de pouvoir être régénéré facilement, et ceci sans que le rendement de la déshydratation, ni la sélectivité en oléfine, diène et polyène respectivement obtenue ne soient affectés. Cette régénération est réalisée par exemple par de l'air, air dilué, enrichi, *in situ* ou *ex situ.* Avantageusement, elle a lieu *in situ.*

La réaction selon l'invention peut être mise en œuvre en phase gazeuse ou en phase liquide, de préférence en phase gazeuse. Lorsque la réaction est menée en phase gazeuse, différentes technologies de procédé peuvent être utilisées, à savoir un procédé en lit fixe, un procédé en lit fluidisé ou un procédé en lit fluidisé circulant, tel que dans un réacteur TZFBR (two zone fluidized bed reactor). Dans les deux premiers procédés, en lit fixe ou en lit fluidisé, la régénération du catalyseur peut être séparée de la réaction catalytique. Elle peut par exemple se faire *ex situ* par les méthodes de régénération conventionnelles, comme la combustion sous air ou avec un mélange gazeux contenant de l'oxygène moléculaire ou un autre oxydant. Selon le procédé de l'invention, la régénération peut se faire *in situ* car les températures et pressions auxquelles se fait la régénération sont compatibles avec les conditions réactionnelles du procédé.

Outre cette régénération visant principalement à éliminer le coke formé à la surface des catalyseurs, une régénération continue ou discontinue du catalyseur peut être conduite visant à renforcer la stabilité à long terme et générer un rapport molaire phosphore sur terres rares (RE) optimum. Pour cela, le catalyseur peut être mis en contact avec un composé à base de phosphore ajouté aux réactifs au cours de la réaction catalytique, de la régénération ou d'une étape dédiée. A titre d'exemples, des composés à base de phosphore appropriés sont choisis parmi le triéthylphosphate ou d'autres alkylphosphates comme le triméthylphosphate, des phosphites comme les triméthylphosphite et triéthylphosphite, et d'autres phosphines. Ces composés peuvent être ajoutés avec ou sans eau; la présence d'un peu d'eau reste préférable.

S'agissant du procédé en phase liquide, la réaction peut être réalisée dans un réacteur classique pour réaction en phase liquide sur un catalyseur solide, mais aussi dans un réacteur de type distillation catalytique eu égard à la différence significative des points d'ébullition des alcools et des oléfines, diènes et polyènes correspondants. On peut également raisonnablement envisager un procédé en phase liquide à une température relativement basse qui permet une distillation continue des produits obtenus, limitant ainsi les réactions consécutives de dégradation.

Les conditions expérimentales de la réaction en phase gazeuse sont de préférence une température comprise entre 150 et 450°C à une pression comprise entre 1 et 10 bars (0.1-1 MPa). En phase liquide, la réaction est opérée entre 50 et 200°C et à une pression pouvant aller de 3 à 70 bars (0.3-7 MPa).

Un autre avantage du procédé de l'invention réside dans la forme des alcools de départ qui peuvent être sous forme pure ou partiellement purifiée ou en solution, notamment aqueuse ou en mélanges. Par ailleurs, les solutions d'alcools ne devront pas être trop diluées, en raison d'un coût énergétique rédhibitoire causé par l'évaporation des alcools. Dans tous les cas, il est pratique d'ajuster la concentration de la solution d'alcool en recyclant partiellement ou totalement l'eau produite par la réaction considérée. L'optimisation énergétique aux bornes de la synthèse tend à récupérer la chaleur en sortie de réaction pour vaporiser le flux d'alcool(s) alimentant le réacteur. Ainsi, dans la suite de la description, on se réfère principalement à la conversion d'un alcool pur ou d'un mélange d'alcools, quels que soient leurs origines et leurs degrés de pureté.

Selon l'utilisation envisagée, il pourra être envisagé de purifier les oléfines, les diènes et polyènes ou le mélange d'oléfines, de diènes et/ou de polyènes obtenu selon les techniques conventionnelles connues de l'homme de l'art.

Un autre objet de l'invention est un procédé de fabrication de l'aldéhyde-3(méthylthio)propionique (MMP), du 2-hydroxy-4-méthylthiobutyronitrile (HMTBN), de la méthionine, de l'acide 2-hydroxy-4-méthylthiobutanoïque (HMTBA), des esters de ce dernier, notamment l'ester isopropylique, et l'acide 2-oxo-4-méthylthiobutanoïque (KMB) à partir de propène, selon lequel le propène est obtenu par un procédé décrit ci-dessus. Le propène subit ensuite une oxydation ménagée en phase gazeuse par l'oxygène de l'air en présence de catalyseurs à base d'oxydes de molybdène et/ou bismuth pour former de l'acroléine.

Après purification, l'acroléine directement obtenue selon l'invention ou après purification est mise en réaction avec du méthylmercaptan (MSH) pour produire l'aldéhyde-3-(méthylthio)propionique (ou MMP). Dans une étape suivante, le MMP est mis en contact avec de l'acide cyanhydrique pour produire le 2-hydroxy-4-(méthylthio)butyronitrile (HMTBN). Après synthèse du HMTBN, diverses étapes réactionnelles conduisent à la méthionine, son hydroxyanalogue (HMTBA), les esters de ce dernier, ou son oxoanalogue (KMB). Toutes ces étapes à compter de la synthèse du propène sont bien connues de l'homme du métier.

A partir de propène, selon lequel le propène est obtenu par un procédé décrit ci-dessus, un autre objet de la présente invention consiste à produire de l'acide acrylique, de l'acrylonitrile et du polypropylène selon les procédés bien connus de l'homme de métier.

L'invention est ci-après illustrée à travers les exemples suivants qui en donnent les détails et les avantages par rapport à l'art antérieur, et à l'appui des figures selon lesquelles :
La Figure 1 représente une comparaison de la conversion de butanol-1, en fonction de la température de réaction pour trois catalyseurs de l'invention et une alumine, dans les conditions décrites à l'exemple 1.
La Figure 2 représente une comparaison de la sélectivité en but-2-ène, en fonction de la température de réaction pour les trois mêmes catalyseurs de l'invention et l'alumine, de la figure 1, dans les conditions décrites à l'exemple 1.
La Figure 3 représente une comparaison de la conversion de butan-1-ol et la sélectivité en but-2-ène, en fonction de la température de réaction pour un catalyseur de l'invention préparé selon deux procédés différents, dans les conditions décrites à l'exemple 2.
La Figure 4 représente l'effet de l'eau sur la déshydratation du butane-2,3-ol en butadiène dans les conditions décrites à l'exemple 15.

Les catalyseurs illustrés sont caractérisés par les paramètres suivants :
- Surface spécifique exprimée en m²/g et mesurée par la méthode BET,
- Teneurs en phosphore et en métal (ou en métaux) M exprimées par un rapport molaire P/M, et mesurées par ICP-OES (spectrométrie d'émission à plasma inductif) ; dans les exemples suivants, ce rapport varie dans la fourchette préférentielle précédemment définie, de 0,9 à 1,5 ; plus précisément, pour les catalyseurs de l'invention testés ci-après, le rapport molaire P/La est de 1,10 pour LaPO₄, et le rapport molaire P/Nd est de 1,14 pour NdPO₄.

La réaction de déshydratation des alcools est conduite sur les catalyseurs indiqués, à pression atmosphérique ou une pression sensiblement supérieure, dans un réacteur à lit fixe. Le réacteur est placé dans un four qui permet de maintenir le catalyseur à la température de réaction qui varie entre 130 et 390°C. Le réacteur est alimenté en alcool à l'aide d'un saturateur ou un pousse-seringue en présence d'un débit d'azote. La proportion relative molaire alcool/azote est indiquée pour chaque exemple. La vitesse spatiale horaire (WHSV) est exprimée en grammes d'alcool introduits par gramme de catalyseur et par heure.

### EXEMPLE 1

Une série d'orthophosphates de terres rares (Nd, Sm, Gd) a été testée dans la réaction de déshydratation du butan-1-ol et comparée à une alumine gamma, pour la conversion du butan-1-ol en but-2-ène.

La surface spécifique de chacun des catalyseurs testés est de 117 m²/g pour NdPO₄, de 82 m²/g pour SmPO₄, de 95 m²/g pour GdPO₄ et de 270 m²/g pour Al₂O₃.

La réaction est conduite à pression atmosphérique dans les conditions suivantes: WHSV =2,38 h⁻¹ ; butan-1-ol/N₂=1/82,6.

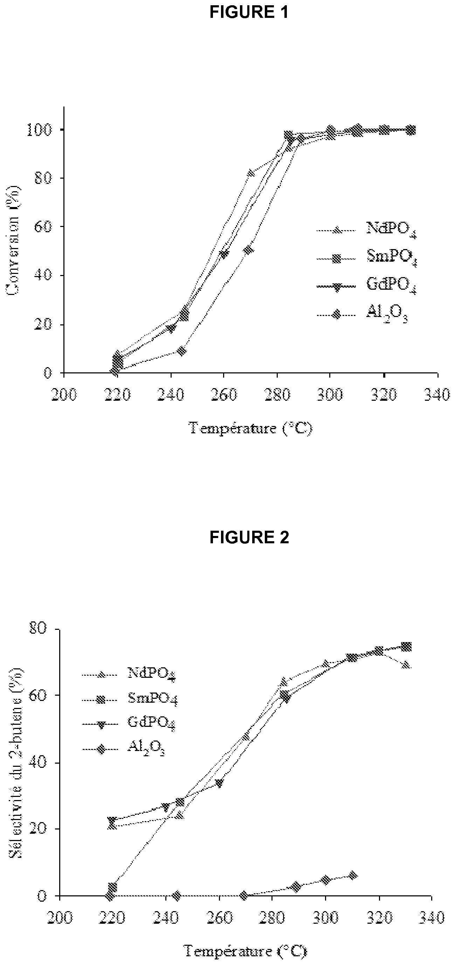

Sur les figures 1 et 2, sont présentées respectivement la conversion du butan-1-ol et la sélectivité en but-2-ène obtenues en fonction de la température de réaction avec la légende suivante :
▲ NdPO₄
■ SmPO₄
▼ GdPO₄
◆ Al₂O₃

Les phosphates apparaissent actifs à plus basse température que l'alumine de référence. Une sélectivité en but-2-ène très supérieure est en outre mesurée.

### EXEMPLE 2

Dans cet exemple, l'orthophosphate de lanthane est préparé à partir de deux précurseurs différents, respectivement, Na₂HPO₄ selon le procédé de fabrication décrit dans J.A. Diaz-Guillen, A.F. Fuentes, S. Gallini, M.T. Colomer, J. All.and Comp. 427 (2007) 87-98, et (NH₄)H₂PO₄ selon la méthode de Pavel.

La surface spécifique de chacun des catalyseurs testés est de 128 m²/g pour LaPO₄ obtenu à partir de Na₂HPO₄ et de 112 m²/g pour LaPO₄ obtenu à partir de (NH₄)H₂PO₄.

Ces catalyseurs ont été testés dans la déshydratation du butan-1-ol dans les conditions de test suivantes: WHSV =2,38 h⁻¹ ; butan-1-ol/N₂ = 1/82,6.

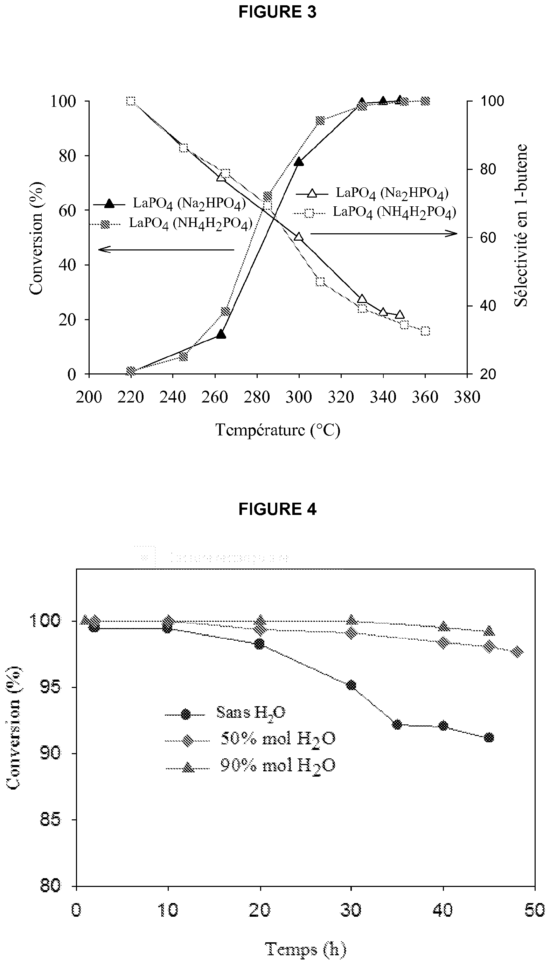

La figure 3 montre l'évolution de la conversion de l'alcool et de la sélectivité en but-1-ène en fonction de la température, avec la légende suivante :
▲ (conversion) et Δ (sélectivité) : LaPO₄ obtenu à partir de Na₂HPO₄
■ (conversion) et □ (sélectivité) : LaPO₄ obtenu à partir de(NH₄)H₂PO₄

On observe que les propriétés catalytiques des catalyseurs de l'invention ne dépendent pas des précurseurs utilisés.

### EXEMPLE 3

Dans cet exemple, l'orthophosphate de néodyme sous deux formes polymorphiques différentes (rhabdophane et monazite) a été testé dans la réaction de déshydratation du butan-1-ol.

Les conditions de test sont les suivantes: WHSV = 2,38 h⁻¹ ; butan-1-ol/N₂ = 1/82,6.

Le tableau 1 montre la conversion de l'alcool et la sélectivité en but-2-ène à 320°C.

**Tableau 1**

| Catalyseur | Conversion du butan-1-ol(%) | Sélectivité en but-2-ène (%) | Sélectivité en but-1-ène (%) |
|---|---|---|---|
| NdPO₄rhabdophane | 99,8 | 73 | 27 |
| NdPO₄ monazite | 99,9 | 72 | 28 |

### EXEMPLE 4

Un mélange 50 : 50 molaire d'éthanol et de butan-1-ol a été déshydraté sur un catalyseur GdPO₄ de l'invention et sur une alumine gamma. Les résultats catalytiques obtenus à 360°C sont présentés dans le tableau 2 ci-dessous.

La surface spécifique du catalyseur de l'invention GdPO₄ est de 95 m²/g et celle de l'alumine est de 270 m²/g.

Les conditions de test sont les suivantes: WHSV = 2,34 h⁻¹ ; N₂ = 100 mL.

Les deux alcools sont totalement transformés sur le catalyseur phosphate. Cette transformation conduit à un mélange éthylène : but-2-ène caractérisé par un rapport molaire de 1,4 qui peut être utilisé directement pour une réaction de métathèse pour former du propène. On constate également que le catalyseur phosphate est très stable.

**Tableau 2**

| Catalyseur | Temps (h) | Conversion de l'éthanol (%) | Conversion du butan-1-ol (%) | Sélectivité en éthylène (%) | Sélectivité en but-2-ène (%) |
|---|---|---|---|---|---|
| GdPO₄ | 1 | 100 | 100 | 100 | 74 |
| | 60 | 99 | 100 | 100 | 71 |
| Al₂O₃ | 1 | 100 | 100 | 99 | 16 |

### EXEMPLE 5 (pas selon cette invention).

Les catalyseurs LaPO₄ et NdPO₄ont été comparés à l'alumine dans la déshydratation du but-3-ène-1-ol.

La surface spécifique des catalyseurs de l'invention NdPO₄et LaPO₄ est de 117 m²/g et de 124 m²/g, respectivement, et celle de l'alumine est de 270 m²/g.

Les conditions réactionnelles sont les suivantes : WHSV = 2,49 h⁻¹ ; 3-but-1-ène-ol/N₂ = 1/76,8.

Le tableau 3 ci-dessous montre la conversion de l'alcool et la sélectivité en butadiène à 286°C.

**Tableau 3**

| Catalyseur | Conversion du 3-but-1-ène-ol (%) | Sélectivité en butadiène (% ) |
|---|---|---|
| NdPO₄ | 100 | 99 |
| LaPO₄ | 92 | 98 |
| Al₂O₃ | 34 | 12 |

### EXEMPLE 6

NdPO₄a été testé et comparé à l'alumine dans la déshydratation de l'isobutanol.

La surface spécifique du catalyseur de l'invention NdPO₄est de 117 m²/g et celle de l'alumine est de 270 m²/g.

Les conditions réactionnelles sont les suivantes : WHSV =2,38 h⁻¹ ; isobutanol/N₂ = 1/82,6.

La conversion de l'isobutanol et la sélectivité en isobutène et en but-2-ène à 245°C, sont données dans le tableau 4 suivant.

**Tableau 4**

| Catalyseur | Conversion de l'isobutanol (%) | Sélectivité en isobutène (%) | Sélectivité en but-2-ène (%) |
|---|---|---|---|
| NdPO₄ | 52 | 91 | 9 |
| Al₂O₃ | 20 | 98 | 2 |

### EXEMPLE 7

NdPO₄a été testé dans la réaction de déshydratation du butan-2-ol et comparé à l'alumine.

La surface spécifique du catalyseur de l'invention NdPO₄est de 117 m²/g et celle de l'alumine est de 270 m²/g.

La réaction est conduite à pression atmosphérique dans les conditions suivantes: WHSV = 2,38 h⁻¹ ; butan-2-ol/N₂ = 1/82,6 ; température du réacteur, 200°C.

**Tableau 5**

| Catalyseur | Conversion du butane-2-ol (%) | Sélectivité en but-2-ène (%) |
|---|---|---|
| NdPO₄ | 76 | 84 |
| Al₂O₃ | 52 | 81 |

### EXEMPLE 8

Le phosphate de néodyme est dans cet exemple testé dans la déshydratation du propan-1-ol.

La surface spécifique du catalyseur de l'invention NdPO₄ est de 117 m²/g.

La réaction conduite à pression atmosphérique dans les conditions suivantes: WHSV = 3,04 h⁻¹ ; propan-1-ol/N₂ = 1/49,5 ; température du réacteur, 330°C.

L'évolution de la conversion du propan-1-ol et de la sélectivité en propène est présentée dans le tableau 6 suivant.

**Tableau 6**

| Catalyseur | Conversion du propan-1-ol (%) | Sélectivité en propène (%) |
|---|---|---|
| NdPO₄ | 99 | 99 |

### EXEMPLE 9 (pas selon cette invention).

Le phosphate de néodyme a été testé comme catalyseur pour la déshydratation du butane-2,3-diol.

La surface spécifique du catalyseur de l'invention NdPO₄ est de 117 m²/g.

La réaction est conduite dans les conditions suivantes :
WHSV =2,95 h⁻¹ ; butane-2,3-ol/N₂ = 1/80,3 ; la température du réacteur est de 320°C.

Les résultats catalytiques obtenus sont présentés dans le tableau 7 suivant.

**Tableau 7**

| Catalyseur | Conversion butane-2,3-diol (%) | Sélectivité en butadiène (%) |
|---|---|---|
| NdPO₄ | 99 | 60 |

Le catalyseur est très actif et sélectif en butadiène.

### EXEMPLE 10 (pas selon cette invention).

Les phosphates de lanthane, de néodyme et de gadolinium ont été testés comme catalyseurs pour la déshydratation du butane-2,3-ol (2,3-BDO).

Les conditions de réaction sont les suivantes : WHSV de 2,98 h⁻¹ ; m_{cata} = 101 mg ; temps de contact (W/F) = 30,28 g_{cata}.h.Mol_{2,3}-_{BDO}⁻¹ ; N₂ = 100 ml.min⁻¹ et un mélange gazeux butane-2,3-ol/N₂ = 1/80,3.

Les résultats catalytiques obtenus sont présentés dans le tableau 8 suivant.

**Tableau 8**

| Catalyseur | Température (°C) | Conversion 2,3-BDO (%) | Sélectivité en butadiène (%) | Sélectivité en MEK (%) | Sélectivité en MPA (%) |
|---|---|---|---|---|---|
| LaPO₄ | 300 | 95,4 | 56 | 7 | 37 |
| NdPO₄ | 320 | 100 | 58 | 7 | 35 |
| GdPO₄ | 300 | 100 | 60 | 7 | 33 |

Les catalyseurs sont très actifs et sélectifs en butadiène.

### EXEMPLE 11 (pas selon cette invention).

Le catalyseur GdPO₄ a été testé dans la déshydratation du 3-butène-2-ol.

Les conditions réactionnelles sont les suivantes : m_{cata} = 101 mg ; temps de contact (W/F) = 28,97 g_{cata}.h.mol⁻¹, 3-butène-2-ol/N₂ = 1/76,8.

Le tableau 9 montre la conversion du 3-butène-2-ol et la sélectivité en butadiène à 230°C.

**Tableau 9**

| Catalyseur | Conversion du 3-butène-2-ol (%) | Sélectivité en butadiène (%) |
|---|---|---|
| GdPO₄ | 100 | 99 |

Le catalyseur est très actif et sélectif en butadiène.

### EXEMPLE 12 (pas selon cette invention).

Le catalyseur GdPO₄ a été testé comme catalyseur pour la déshydratation du but-3-ène-1,2-diol.

Les conditions réactionnelles sont les suivantes: m_{cata} = 118 mg ; temps de contact (W/F) = 30,3 g_{cata}.h.mol⁻¹ ; Alcool/N₂= 1/68 ; T = 310°C.

Les résultats catalytiques obtenus sont présentés dans le tableau 10 suivant.

**Tableau 10**

| Catalyseur | Conversion du 3-butène 1,2-ol (%) | Sélectivité en butenal (%) | Sélectivité en butadiène (%) | Sélectivité en autres produits^{*} (%) |
|---|---|---|---|---|
| GdPO₄ | 96 | 98 | 1 | 1 |

| | | | | |
|---|---|---|---|---|
| * Autres produits : méthyl vinyl cétone, 1,3-butadiénol, 2,5-dihydrofurane | | | | |

### EXEMPLE 13

SmPO₄ a été testé dans la réaction de déshydratation conjointe de l'éthanol, du propanol et du butanol.

La réaction est conduite à pression atmosphérique dans les conditions suivantes:
Temps de contact (W/F) = 28,33 g_{cata}.h.mol⁻¹,
Ethanol/1-propanol/2-propanol/2-butanol = 10/15/15/60,
N2 = 100 mL.min⁻¹ ;
Température du réacteur : 330°C.

Comme indiqué dans le tableau 11 suivant, on observe à cette température, 100% de conversion de tous les alcools avec pratiquement 100% de sélectivité en alcènes correspondants.

**Tableau 11**

| Catalyseur | Conversion de l'éthanol (%) | Sélectivité en éthylène (%) | Conversion du 1- et 2-propanol (%) | Sélectivité en propène (%) | Conversion du 2-butanol (%) | Sélectivité en 1-/2-Butène (%) |
|---|---|---|---|---|---|---|
| NdPO₄ | 99,1 | 100 | 100 | 99.5 | 100 | 27/73 |
| SmPO₄ | 100 | 100 | 100 | 100 | 100 | 24/76 |

### EXEMPLE 14

SmPO₄ et NdPO₄ ont été étudié dans la déshydratation d'un mélange ABE (acétone/butanol/éthanol) issu de fermentation.

Le test est conduit à pression atmosphérique dans les conditions suivantes:
m_{cata} = 1 01 mg ;
Temps de contact (W/F) = 28,1 g_{cata}.h.mol⁻¹,
Acétone/1 -butanol/éthanol = 3/6/1,
N₂ = 100 ml.min⁻¹ ;
Température du réacteur = 330°C.

Le tableau 12 suivant présente les résultats.

**Tableau 12**

| Catalyseur | Conversion de l'acétone (%) | Conversion de l'éthanol (%) | Sélectivité en éthylène (%) | Conversion du 1-Butanol (%) | Sélectivité en 1-/2-Butène (%) |
|---|---|---|---|---|---|
| NdPO₄ | 0 | 100 | 100 | 100 | 34/66 |
| SmPO₄ | 0 | 100 | 100 | 100 | 30/70 |

### EXEMPLE 15 (pas selon cette invention)

Quand il est produit par fermentation, le butane-2,3-ol (2,3-BDO) est en mélange avec une quantité importante d'eau qui doit être éliminée par évaporation. Si cette séparation peut être totalement ou partiellement évitée avant l'étape de déshydratation en butadiène, cela représenterait un intérêt économique certain.

Le phosphate de gadolinium a été testé en présence d'eau.

La réaction est conduite dans les conditions suivantes:
Température = 300°C ; m_{cata}=101 mg ; temps de contact (W/F) = 30,28 g_{cata}.h.Mol_{2,3}-_{BDO}⁻¹ ; N₂ = 100 ml.min⁻¹

Les résultats sont présentés dans le tableau 13 suivant et illustrés sur la figure 4.

**Tableau 13**

| Catalyseur | Eau (% mol) | Conversion butane2,3-ol (%) | Sélectivité en butadiène (%) | Sélectivité en MEK (%) | Sélectivité en MPA (%) |
|---|---|---|---|---|---|
| GdPO₄ | 0 | 100 | 60 | 7 | 33 |
| | 50 | 100 | 50 | 10 | 40 |
| | 90 | 100 | 43 | 13 | 44 |

On observe qu'une augmentation de la quantité d'eau ne modifie pas significativement les propriétés catalytiques et baisse seulement légèrement la sélectivité en butadiène.

L'effet de l'eau sur la stabilité du catalyseur en conditions réactionnelles a aussi été étudié. L'eau a un effet très positif sur cette stabilisation.

### EXEMPLE 16

Un mélange 50:50 molaire d'éthanol et de 1-butanol a été déshydraté sur des phosphates de gadolinium, de samarium et de néodyme.

Les conditions de réaction catalytiques sont :
m_{cata}: 101 mg, temps de contact (W/F) = 25 g_{cata}.h.mol⁻¹, N₂ = 100 mL.min⁻¹

Les résultats catalytiques obtenus sont présentés dans le tableau 14 ci-dessous.

**Tableau 14**

| Catalyseur | Température (°C) | Conversion de l'éthanol (%) | Conversion du 1-butanol (%) | Sélectivité en éthylène (%) | Sélectivité en 2-butène (%) |
|---|---|---|---|---|---|
| GdPO₄ | 360 | 100 | 100 | 100 | 74 |
| SmPO₄ | 360 | 99,8 | 100 | 100 | 75 |
| NdPO₄ | 350 | 97,8 | 100 | 100 | 73 |

Les deux alcools sont totalement transformés sur les catalyseurs. Les catalyseurs sont très actifs et sélectifs pour les deux déshydratations. Le catalyseur SmPO₄ est le plus sélectif pour la formation de 2-butène.

## Revendications

1. Procédé de préparation du propène, comprenant la préparation d'une oléfine ou un mélange de deux oléfines, par conversion catalytique d'au moins un alcool possédant une chaîne carbonée d'au moins trois atomes de carbone et différent du propan-2-ol, en présence d'au moins un catalyseur à base d'au moins un phosphate d'un métal ou de plusieurs métaux M, M étant choisi parmi le lanthane, le praséodyme, le néodyme, le prométhium, le samarium, l'europium, le gadolinium, le terbium, le dysprosium, l'holmium, l'erbium, le thulium, l'ytterbium, le lutécium, l'yttrium et le scandium, procédé selon lequel,
- on prépare le propène par ladite conversion catalytique d'un alcool choisi parmi le propan-1-ol, éventuellement en mélange avec le propan-2-ol ;
- on prépare un butène par ladite conversion catalytique d'un alcool choisi parmi le butan-1-ol, le butan-2-ol et leurs mélanges, puis on convertit le butène en propène par métathèse ; ou
- on prépare un mélange éthylène et butène par ladite conversion catalytique de l'éthanol et d'au moins un autre alcool choisi parmi le butan-1-ol, le butan-2-ol et leurs mélanges, puis on convertit le mélange éthylène et butène en propène par métathèse.

2. Procédé selon la revendication 1, **caractérisé en ce que** le(s) métal(aux) sont choisis parmi le lanthane, le néodyme, le gadolinium et le samarium.

3. Procédé selon la revendication 1 ou 2, **caractérisé en ce que** ledit catalyseur est dopé par au moins un métal alcalin choisi parmi le césium, le rubidium et le potassium et/ou un métal de transition choisi dans les groupes 4 à 7.

4. Procédé selon l'une quelconque des revendications 1 à 3, **caractérisé en ce que** le rapport molaire P/M (teneurs en phosphore et en métal (ou en métaux) exprimées par un rapport molaire P/M mesurées par spectrométrie d'émission à plasma inductif) varie de 0,5 à 2, M représentant le métal ou la somme des métaux M constitutifs dudit catalyseur.

5. Procédé selon la revendication 4, caractérisé en ce le rapport molaire P/M (teneurs en phosphore et en métal (ou en métaux) exprimées par un rapport molaire P/M mesurées par spectrométrie d'émission à plasma inductif) varie de 0,9 à 1,5.

6. Procédé selon l'une quelconque des revendications 1 à 5, **caractérisé en ce que** le catalyseur comprend une phase active et un support et/ou un liant et/ou le catalyseur est mis en forme, éventuellement en présence d'un liant, le support et/ou le liant étant constitués de silice pure (SiO₂), d'un silicate d'alcalin, d'alcalino-terreux ou de terres rares, éventuellement en mélange entre eux ou avec les argiles, d'oxyde de titane (TiO₂), d'oxyde de bore (B₂O₃) ou des résines (sulphoniques, perfluorosulphoniques ou autres) et de leurs mélanges.

7. Procédé selon l'une des revendications 1 à 6, **caractérisé en ce que** la conversion est réalisée en phase gazeuse ou en phase liquide.

8. Procédé selon l'une quelconque des revendications 1 à 6, **caractérisé en ce que** l'alcool ou les alcools sont en solution aqueuse, en une concentration d'au moins 1% en poids.

9. Procédé selon l'une quelconque des revendicaions 1 à 8, **caractérisé en ce que** l'éthylène et le butène sont préparés dans le même réacteur ou dans des réacteurs différents.

10. Procédé selon l'une quelconque des revendications 1 à 9, caractérisé en ce les catalyseurs pour la conversion catalytique de l'éthylène et du butène respectivement sont identiques ou différents.

11. Procédé de fabrication de l'acroléine, de l'acide acrylique et de l'acrylonitrile, du polypropylène à partir de propène, **caractérisé en ce qu'**il met en œuvre un procédé selon l'une quelconque des revendications 1 à 10.

12. Procédé de fabrication de l'aldéhyde-3-(méthylthio)propionique MMP, du 2-hydroxy-4-méthylthiobutyronitrile (HMTBN), de la méthionine, de l'acide 2-hydroxy-4-méthylthiobutanoïque (HMTBA), des esters de ce derniers, ou du 2-oxo-4-méthylthiobutanoïque (KMB), à partir d'acroléine, **caractérisé en ce qu'**il met en œuvre un procédé selon la revendication 11.

## Patentansprüche

1. Verfahren zur Herstellung von Propen, umfassend die Herstellung eines Olefins oder eines Gemisches aus zwei Olefinen durch katalytische Umwandlung von mindestens einem Alkohol, der eine Kohlenstoffkette mit mindestens drei Kohlenstoffatomen besitzt und sich von Propan-2-ol unterscheidet, in Gegenwart von mindestens einem Katalysator auf der Grundlage von mindestens einem Phosphat eines Metalls oder mehrerer Metalle M, wobei M ausgewählt ist aus Lanthan, Praseodym, Neodym, Promethium, Samarium, Europium, Gadolinium, Terbium, Dysprosium, Holmium, Erbium, Thulium, Ytterbium, Lutecium, Yttrium und Scandium, Verfahren, bei dem
- das Propen durch die katalytische Umwandlung von einem Alkohol hergestellt wird, der ausgewählt ist aus Propan-1-ol, eventuell im Gemisch mit Propan-2-ol;
- ein Buten durch die katalytische Umwandlung von einem Alkohol hergestellt wird, der ausgewählt ist aus Butan-1-ol, Butan-2-ol und deren Gemischen, und das Buten danach durch Metathese in Propen umgewandelt wird; oder
- ein Gemisch aus Ethylen und Buten durch die katalytische Umwandlung von dem Ethanol und mindestens einem anderen Alkohol hergestellt wird, der ausgewählt ist aus Butan-1-ol, Butan-2-ol und deren Gemischen und das Gemisch aus Ethylen und Buten danach durch Metathese in Propen umgewandelt wird.

2. Verfahren nach Anspruch 1, **dadurch gekennzeichnet, dass** das (die) Metall(e) ausgewählt ist (sind) aus Lanthan, Neodym, Gadolinium und Samarium.

3. Verfahren nach Anspruch 1 oder 2, **dadurch gekennzeichnet, dass** der Katalysator mit mindestens einem alkalischen Metall dotiert ist, ausgewählt aus Cäsium, Rubidium, und Kalium und/oder einem Übergangsmetall ausgewählt aus den Gruppen 4 bis 7.

4. Verfahren nach einem der Ansprüche 1 bis 3, **dadurch gekennzeichnet, dass** das P/M-Molverhältnis (Gehalt an Phosphor und an Metall (oder an Metallen), ausgedrückt durch ein durch Induktionsplasma-Emissionsspektrometrie gemessenes P/M-Molverhältnis) von 0,5 bis 2 variiert, wobei M das Metall oder die Summe der Metalle M repräsentiert, aus denen der Katalysator besteht.

5. Verfahren nach Anspruch 4, **dadurch gekennzeichnet, dass** das P/M-Molverhältnis (Gehalt an Phosphor und an Metall (oder an Metallen), ausgedrückt durch ein durch Induktionsplasma-Emissionsspektrometrie gemessenes P/M-Molverhältnis) von 0,9 bis 1,5 variiert.

6. Verfahren nach einem der Ansprüche 1 bis 5, **dadurch gekennzeichnet, dass** der Katalysator eine aktive Phase und einen Träger und/oder ein Bindemittel umfasst, und/oder der Katalysator eventuell in Gegenwart eines Bindemittels in Form gebracht wird, wobei der Träger und/oder das Bindemittel aus reinem Silicium (SiO₂), einem Alkali-, Erdalkali- oder aus seltenen Erden bestehenden Silikat, eventuell im Gemisch miteinander oder mit den Lehmen, aus Titanoxid (TiO₂), Boroxid (B₂O₃) oder (sulfonischen, perfluorsulfonischen oder anderen) Harzen und deren Gemischen gebildet sind.

7. Verfahren nach einem der Ansprüche 1 bis 6, **dadurch gekennzeichnet, dass** die Umwandlung in Gasphase oder Flüssigphase ausgeführt wird.

8. Verfahren nach einem der Ansprüche 1 bis 6, **dadurch gekennzeichnet, dass** der Alkohol oder die Alkohole in wässriger Lösung mit einer Konzentration von mindestens 1 Gew.-% sind.

9. Verfahren nach einem der Ansprüche 1 bis 8, **dadurch gekennzeichnet, dass** das Ethylen und das Buten im selben Reaktor oder in unterschiedlichen Reaktoren hergestellt werden.

10. Verfahren nach einem der Ansprüche 1 bis 9, **dadurch gekennzeichnet, dass** die Katalysatoren zur katalytischen Umwandlung vom Ethylen und Buten jeweils identisch oder unterschiedlich sind.

11. Verfahren zur Herstellung von Acrolein, Acrylsäure und Acrylnitril, Polypropylen aus Propen, **dadurch gekennzeichnet, dass** es ein Verfahren nach einem der Ansprüche 1 bis 10 anwendet.

12. Verfahren zur Herstellung von 3-(Methylthio)Propionaldehyd MMP, 2-Hydroxy-4-Methylthiobutyronitril (HMTBN), Methionin, 2-Hydroxy-4-Methylthiobutansäure (HMTBA), von Estern dieser letzteren, oder 2-Oxo-4-Methylthiobutansäure (KMB) aus Acrolein, **dadurch gekennzeichnet, dass** es ein Verfahren nach Anspruch 11 anwendet.

## Claims

1. A method for preparing propene, comprising the preparation of an olefin or a mixture of two olefins, by catalytic conversion of at least one alcohol having a carbon chain of at least three carbon atoms and different from propan-2-ol, in the presence of at least one catalyst based on at least one phosphate of one metal or several metals M, M being selected from lanthanum, praseodymium, neodymium, promethium, samarium, europium, gadolinium, terbium, dysprosium, holmium, erbium, thulium, ytterbium, lutetium, yttrium and scandium, the method according to which,
- propene is prepared by said catalytic conversion of an alcohol selected from propan-1-ol, optionally mixed with propan-2-ol;
- a butene is prepared by said catalytic conversion of an alcohol selected from butan-1-ol, butan-2-ol and their mixtures, then the butene is converted into propene by metathesis; or
- an ethylene and butene mixture is prepared by said catalytic conversion of ethanol and at least one other alcohol selected from butan-1-ol, butan-2-ol and their mixtures, then the ethylene and butene mixture is converted to propene by metathesis.

2. The method according to claim 1, **characterized in that** the metal(s) are selected from lanthanum, neodymium, gadolinium and samarium.

3. The method according to claim 1 or 2, **characterized in that** said catalyst is doped with at least one alkali metal selected from cesium, rubidium and potassium and/or a transition metal selected from the groups 4 to 7.

4. The method according to any one of claims 1 to 3, **characterized in that** the molar ratio P/M (contents of phosphorus and metal (or metals) expressed by a molar ratio P/M measured by inductive plasma emission spectrometry) varies from 0.5 to 2, M representing the metal or the sum of the metals M constituting said catalyst.

5. The method according to claim 4, **characterized in that** the molar ratio P/M (contents of phosphorus and metal (or metals) expressed by a molar ratio P/M measured by inductive plasma emission spectrometry) varies from 0.9 to 1.5.

6. The method according to any one of claims 1 to 5, **characterized in that** the catalyst comprises an active phase and a support and/or a binder and/or the catalyst is shaped, optionally in the presence of a binder, the support and/or the binder consisting of pure silica (SiO₂), of an alkali, alkaline earth or rare earth silicate, optionally mixed with one another or with the clays, of titanium oxide (TiO₂), boron oxide (B₂O₃) or resins (sulphonic, perfluorosulphonic or other) and their mixtures.

7. The method according to any of claims 1 to 6, **characterized in that** the conversion is carried out in the gas phase or in the liquid phase.

8. The method according to any one of claims 1 to 6, **characterized in that** the alcohol or alcohols are in aqueous solution, in a concentration of at least 1% by weight.

9. The method according to any one of claims 1 to 8, **characterized in that** the ethylene and butene are prepared in the same reactor or in different reactors.

10. The method according to any one of claims 1 to 9, **characterized in that** the catalysts for the catalytic conversion of ethylene and butene respectively are identical or different.

11. The method for manufacturing acrolein, acrylic acid and acrylonitrile, polypropylene from propene, **characterized in that** it implements a method according to any one of claims 1 to 10.

12. The method for manufacturing aldehyde-3 (methylthio) propionic MMP, 2-hydroxy-4-methylthiobutyronitrile (HMTBN), methionine, 2-hydroxy-4-methylthiobutanoic acid (HMTBA), esters of the latter, or of 2-oxo-4-methylthiobutanoic acid (KMB), from acrolein, **characterized in that** it implements a method according to claim 11.
